# EUROPEAN PATENT SPECIFICATION

(11) **EP 3 266 411 B1**
(45) Date of publication and mention of the grant of the patent: **26.06.2019**
(21) Application number: 16758709.6
(22) Date of filing: 05.02.2016
(51) Int. Cl.: A61C 19/00, A61C 8/00, A61L 2/10

(54) **ULTRAVIOLET IRRADIATION DEVICE FOR IMPLANT**
ULTRAVIOLETTBESTRAHLUNGSVORRICHTUNG FÜR IMPLANTAT
DISPOSITIF D'IRRADIATION PAR ULTRAVIOLETS POUR UN IMPLANT

(30) Priority: 05.03.2015 JP 2015043569
(43) Date of publication of application: 10.01.2018
(73) Proprietor: Ushio Denki Kabushiki Kaisha, Tokyo 100-8150 (JP)
(72) Inventor: KOBAYASHI Yusuke, Himeji-shi Hyogo 671-0224 (JP)
(74) Representative: Tomerius, Isabel
(86) International application number: PCT/JP2016/053493
(87) International publication number: WO 2016/140014

(56) References cited:
- WO-A1-2012/105093
- WO-A1-2012/117903
- JP-A- H09 327 502
- JP-A- 2004 121 577
- JP-A- 2012 157 413
- US-A1- 2013 189 642
- US-A1- 2013 264 495

## Description

### TECHNICAL FIELD

The present invention relates to an ultraviolet irradiation device for an implant, which is adapted to irradiate the implant with ultraviolet beams and remove organic contaminants from the implant. In particular, the present invention relates to an ultraviolet irradiation device for an implant, which utilizes an ultraviolet lamp to remove organic contaminants from a dental implant.

### BACKGROUND ART

An implant, in particular, a dental implant is buried in a skeleton (skeleton bones) in place of a lost tooth such that the implant functions as a replacement tooth. In a dental implant surgery, it is very important for the dental implant to sufficiently connect to a bone (Osseointegration). In this context, WO 2012/105093 A1 discloses a device for sterilizing dental implants comprising an UV irradiation lamp, a carriage and a retainer. The carriage with the retainer holding the implants is displaced to a position between the UV lamps so as to be placed within an effective emitting region. Japanese Patent No. 3072303 (Patent Literature Document 1), paragraph 0009 also proposes a technique that irradiates a dental implant with an ultraviolet beam to remove organic contaminants that adhere to the dental implant.

As a concrete device to realize this technique, developed is a device that is disclosed in Japanese Patent Application Laid-Open Publication No. 2012-157413 (Patent Literature Document 2).

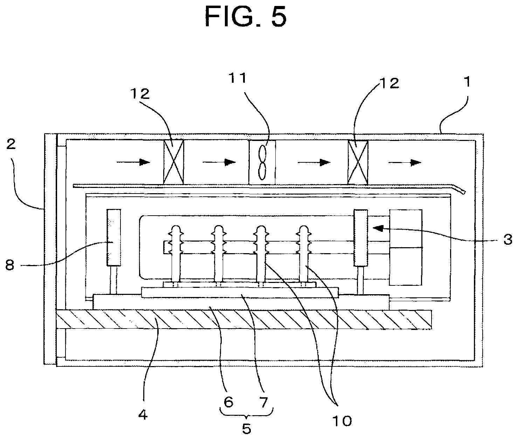

Fig. 5 of the accompanying drawings shows a conventional ultraviolet irradiation device for an implant, which is disclosed in the Document 2.

This ultraviolet irradiation device for the implant includes a housing 1, which has an openable door 2, and a pair of ultraviolet lamps 3 and 3 disposed in the housing 1. A pull-out carriage 4, which is united to the door 2, is disposed in the housing 1. An implant retainer or holder 5 is disposed on the carriage 4.

The implant retainer 5 has a support table or base 6, which is placed on the carriage 4 and is detachable from the carriage 4. The implant retainer 5 also has an implant support 7, which is placed on the support table 6 and detachable from the support table 6.

The support table 6 has handles 8, and a plurality of implants 10 and 10, which are objects to be treated, are linearly arranged on the implant support 7 along the longitudinal direction of the ultraviolet lamps 3. Having the above-described configuration, the support table 6 with the handles 8 being provided thereon becomes an unclean area, and the implant support 7 with the implants 10 being provided thereon becomes a clean area and is distinguished from the unclean area.

In this manner, the implant retainer 5 is divided into the clean area and the unclean area, and the support table 6 (unclean area) is used to retract and extend (push in and pull out) the implant retainer 5. Thus, hands do not contact the implants 10 on the implant support 7, which is located in the clean area, and the implants are kept clean. This configuration is, therefore, not re-contaminated after the ultraviolet irradiation treatment.

With this ultraviolet irradiation device for the implant, if the implant retainer 5 having the implants 10 thereon is placed on the carriage 4, the carriage 4 is received in the housing 1, and the door 2 is closed, then the implants 10 linearly arranged on the implant retainer 5 are situated between a pair of ultraviolet lamps 3 and 3 such that the implants face the ultraviolet lamps.

The implants 10 are irradiated with ultraviolet beams from the ultraviolet lamps 3 at a wavelength of, for example, 172nm or 254nm for a predetermined period. After the ultraviolet irradiation treatment, a fan 11 rotates to circulate the gas in the housing 1, and an ozone removal treatment is carried out by ozone removing units 12.

Each of the ultraviolet lamps used in the above-described ultraviolet irradiation device for the implant has a particular illuminance distribution. Also, a protecting cover may be provided in the housing to prevent fragments of the ultraviolet lamps from spreading when the ultraviolet lamps break. In order to remove organic substances from the implants in an effective manner, each of various devices needs a certain intensity of ultraviolet irradiation, but such intensity is only obtained in a limited area (effective irradiation area) even in the treatment chamber.

In general, implants have various shapes. At an actual site of use of this ultraviolet irradiation device (medical facility or a medical institution), various shapes of implants are simultaneously used on a daily basis.

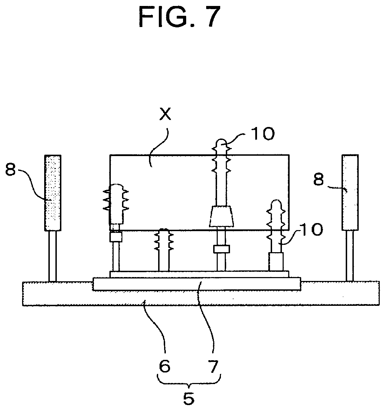

Under such circumstances, as shown in Fig. 7, for example, implants having different lengths may be arranged on the implant support 7. Part of a long implant (upper part thereof) may extend beyond the effective irradiation area X, and a short implant may not reach the effective irradiation area X.

When a plurality of implants are linearly arranged, two of them, which are situated on opposite ends, may be situated outside the effective irradiation area X.

As described above, some of the implants arranged on the implant support may be situated outside the effective irradiation area, and the ultraviolet irradiation treatment may be carried out without recognizing such fact. Then, there is a problem that a user may believe that the organic substance removal treatment has been applied to all of the implants although it is not true.

The conventional device has no mechanism to confirm whether or not the implants, which are the objects to be treated, are present in the effective irradiation area. Thus, the above-described problem is serious.

### LISTING OF REFERENCES

### PATENT LITERATURE DOCUMENTS

Patent Literature Document 1: Japanese Patent No. 3072303
Patent Literature Document 2: Japanese Patent Application Laid-Open Publication No. 2012-157413

### SUMMARY OF THE INVENTION

### PROBLEMS TO BE SOLVED BY THE INVENTION

An object of the present invention is to provide an ultraviolet irradiation device for an implant or implants, which is adapted to remove an organic contaminant from the implant(s) by an ultraviolet beam, and which includes an ultraviolet lamp disposed in a housing, a carriage disposed in the housing such that the carriage can be pulled out from the housing, and an implant retainer disposed on the carriage such that the implant retainer faces the ultraviolet lamp in the housing, with a configuration to determine in advance whether the implant(s) retained on the implant retainer is (are) present inside an effective irradiation area of an ultraviolet beam emitted from the ultraviolet lamp, whereby the organic contaminant is completely removed from all of the implants.

### SOLUTION TO THE PROBLEMS

In order to achieve the above-mentioned object, the implant retainer of the ultraviolet irradiation device for implants according to an aspect of the present invention has an effective irradiation area confirming unit that is configured to determine an effective irradiation area of an ultraviolet beam emitted from the ultraviolet lamp.

The implant retainer may include an implant support on which a plurality of implants are arranged linearly, and a support table on which the implant support is detachably disposed. The effective irradiation area confirming unit may include an index or indices formed on the implant retainer to define a range of the effective irradiation area in a longitudinal direction of the effective irradiation area, and another index or indices formed on a handle provided on the support table to define a length of a range of the effective irradiation area in a vertical direction of the effective irradiation area.

The index (or indices) of the effective irradiation area confirming unit that defines (or define) the range of the effective irradiation area in the longitudinal direction of the effective irradiation area may be a length of a structural element of the implant support in the longitudinal direction of the effective irradiation area.

The effective irradiation area confirming unit may be a plate member that is detachably attached to the support table, and the plate member may have an opening formed therein that corresponds to the effective irradiation area.

The effective irradiation area confirming unit may be a plate member that is detachably attached to the support table and that transmits light, and the plate member may have an index (or indices) formed thereon that corresponds (correspond) to the effective irradiation area.

### ADVANTAGEOUS EFFECTS OF THE INVENTION

The ultraviolet irradiation device for the implant(s) according to the present invention has the effective irradiation area confirming unit to confirm (determine) the effective irradiation area of the ultraviolet beams emitted from the ultraviolet lamp(s). Therefore, it is possible to determine, prior to the ultraviolet irradiation treatment, whether or not all of the implants arranged on the implant support are present inside the effective irradiation area. As such, the organic contaminant removal from the implants is completely carried out without exception.

### BRIEF DESCRIPTION OF THE DRAWINGS

Fig. 1 is a front view of major parts of an ultraviolet irradiation device for implants according to one embodiment of the present invention.
Fig. 2 is a front cross-sectional view of a second embodiment.
Fig. 3 is a front cross-sectional view of a third embodiment.
Fig. 4A is a front view of a fourth embodiment.
Fig. 4B is a side view of the fourth embodiment.
Fig. 5 is a cross-sectional view of an ultraviolet irradiation device for implants according a conventional technology.

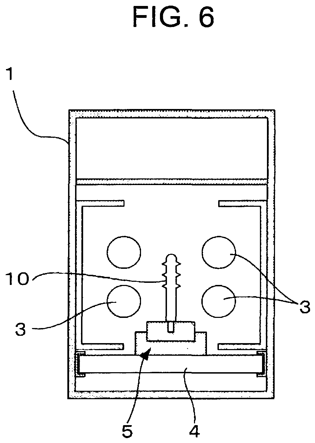

Fig. 6 is a transverse cross-sectional view of Fig. 5.
Fig. 7 is a view useful to describe problems of a conventional device.

### DESCRIPTION OF EMBODIMENTS

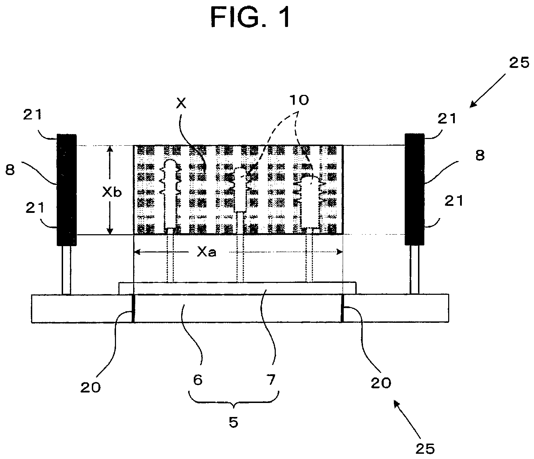

As illustrated in Fig. 1, an ultraviolet irradiation device for implants according to an embodiment of the present invention has longitudinal indices or signs 20 on a longitudinal side face 6a of a support table 6 of an implant retainer 5 such that the longitudinal indices are present at positions that correspond to the range (length) Xa of an effective irradiation area X, which is basically decided by ultraviolet lamps, in a longitudinal direction. The indices 20 may be marks made by appropriate painting, or grooves made by carving.

In this specification, the longitudinal direction is a direction along a linear arrangement direction of the implants 10 in Fig. 5, and is also an extending direction of each of the ultraviolet lamps 3.

On each of the handles 8 provided on the support table 6, formed are vertical indices 21 such that the vertical indices are present at positions that correspond to the vertical range (length) Xb of the effective irradiation area X.

The longitudinal indices 20 and the vertical indices 21 define, in combination, the effective irradiation area X, and serve as an effective irradiation area confirming unit 25.

With such unit, it is possible to confirm whether all of the implants 10, which are objects to be treated, are situated inside the effective irradiation area X.

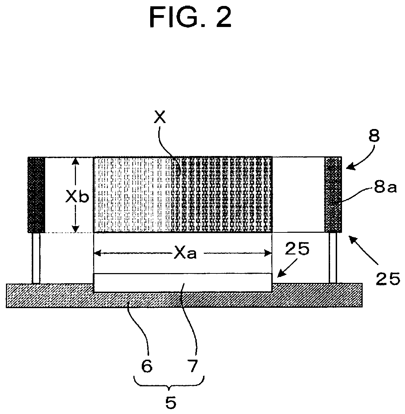

Fig. 2 illustrates a second embodiment in which the indices 20 and 21 are made by different configurations. The longitudinal indices 20 are given by the entire longitudinal length of the implant support 7 placed on the support table 6.

The vertical indices 21 are given by the entire length of a gripping portion 8a of the handle 8.

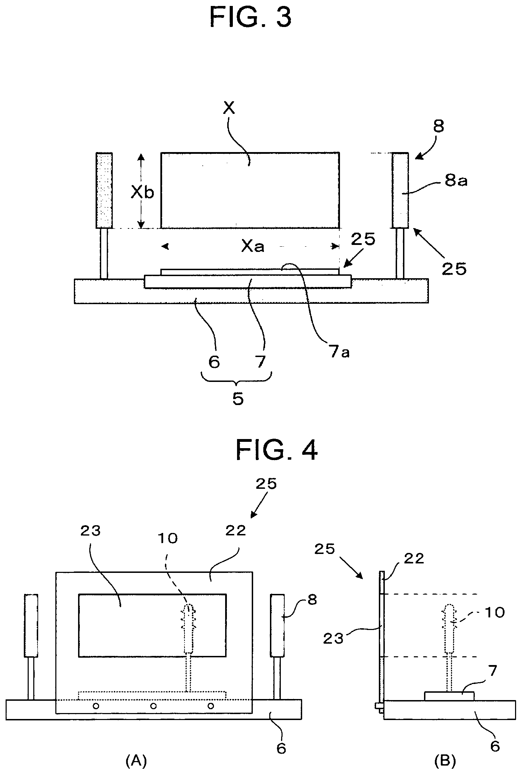

In a third embodiment shown in Fig. 3, the longitudinal indices 20 are given by a longitudinal length of a step portion 7a formed on the implant support 7.

In each of the second embodiment shown in Fig. 2 and the third embodiment shown in Fig. 3, the dimension of the structural element of the implant support 7 in the longitudinal direction is equal to the longitudinal length Xa of the effective irradiation area X.

In each of the above-described embodiments, the implants 10 are arranged on the implant support 7 of the implant retainer 5 prior to the ultraviolet irradiation treatment. Then, the implant retainer is placed on the support table 6, and the effective irradiation area X is defined by the effective irradiation area confirming unit 25 that includes the longitudinal indices 20 and the vertical indices 21. Subsequently, it is confirmed whether all of the implants 10 are present inside the effective irradiation area X. After confirming a fact that all of the implants 10 are present inside the effective irradiation area X, the implant retainer 5 is received in the housing, and the ultraviolet irradiation treatment is carried out.

Fig. 4 shows still another embodiment, i.e., a fourth embodiment. Specifically, Fig. 4(A) is a front view of the fourth embodiment, and Fig. 4(B) is a side view of the fourth embodiment.

In this embodiment, the effective irradiation area confirming unit 25 is a plate member 22. The plate member 22 has an opening 23 that corresponds to the effective irradiation area X. The plate member 22 is attached to the support table 6, and is detachable from the support table 6.

In this embodiment, the implant support 7 on which the implant 10 is arranged is placed on the support table 6 prior to the ultraviolet irradiation treatment. Then, the plate member 22 is attached to the support table 6, and it is confirmed whether or not the implant 10 is present in the effective irradiation area X defined by the opening 23 of the plate member.

After confirming a fact that the implant 10 is present in the effective irradiation area X, the plate member 22 is detached (removed) from the support table 6, the implant retainer 5 is received in the housing, and the ultraviolet irradiation treatment is carried out.

It should be noted that in the fourth embodiment the plate member 22 may be a plate member that transmits light, and indices that correspond to the effective irradiation area X may be formed in (or on) such plate member. Similar to the above-described indices, such indices may be made by drawing with paints or by groove carving.

As described above, the implant retainer of the ultraviolet irradiation device for the implant(s) according to the present invention has the effective irradiation area confirming unit to confirm (determine) the effective irradiation area of the ultraviolet beams emitted from the ultraviolet lamps. Therefore, it is possible, prior to the ultraviolet irradiation treatment, to determine whether or not the implant(s) is (are) present inside the effective irradiation area of the ultraviolet lamps upon arranging the implant(s) on the implant support. As such, when the implant(s) on the implant support is (are) received in the housing, and situated at a position (positions) that correspond(s) to the ultraviolet lamps, all of the implants are irradiated with the ultraviolet beams without exception, and the removal of the organic contaminants is completely carried out.

### REFERENCE NUMERALS AND SYMBOLS

1: Housing
2: Openable door
3: Ultraviolet lamp
4: Carriage
5: Implant retainer
6: Support table
7: Implant support
8: Handle
10: Implant
20: Longitudinal index
21: Vertical index
22: Plate member
23: Opening
25: Effective irradiation area confirming unit
X: Effective irradiation area
Xa: Range (length) of the effective irradiation area in the longitudinal direction
Xb: Range (length) of the effective irradiation area in the vertical direction

## Claims

1. An ultraviolet irradiation device for implants (10), adapted to remove an organic contaminant from the implants by an ultraviolet beam, comprising:
an ultraviolet lamp (3) disposed in a housing (1);
a carriage (4) disposed in the housing such that the carriage can be pulled out from the housing; and
an implant retainer (5) disposed on the carriage such that the implant retainer faces the ultraviolet lamp in the housing,
the implant retainer (5) including an implant support (7) on which the implants (10) are linearly arranged, and a support table (6) on which the implant support is detachably disposed,
**characterized by**
the implant retainer including an effective irradiation area confirming unit (25) that is configured to determine an effective irradiation area (X) of an ultraviolet beam emitted from the ultraviolet lamp,
the effective irradiation area confirming unit (25) including:
a first index (20) formed on the implant retainer and configured to define a range (Xa) of the effective irradiation area (X) in a longitudinal direction of the effective irradiation area; and
a second index (21) formed on a handle (8) provided on the support table and configured to define a range (Xb) of the effective irradiation area in a vertical direction of the effective irradiation area.

2. The ultraviolet irradiation device for implants according to claim 1, wherein the first index (20) of the effective irradiation area confirming unit configured to define the range (Xa) of the effective irradiation area in the longitudinal direction of the effective irradiation area is a length of a structural element (7; 7a) of the implant support in the longitudinal direction of the effective irradiation area (X).

3. The ultraviolet irradiation device for implants according to claim 1, wherein the effective irradiation area confirming unit is a plate member (22) that is detachably attached to the support table, and the plate member has an opening (23) formed therein that corresponds to the effective irradiation area (X).

4. The ultraviolet irradiation device for implants according to claim 1, wherein the effective irradiation area confirming unit is a plate member (22) that transmits light and is detachably attached to the support table (6), and the plate member has an index formed thereon that corresponds to the effective irradiation area (X).

## Patentansprüche

1. UV-Bestrahlungseinrichtung für Implantate (10), die geeignet ist, durch einen UV-Lichtstrahl eine organische Verunreinigung von den Implantaten zu entfernen, umfassend:
eine in einem Gehäuse (1) angeordnete UV-Lampe (3),
einen Schlitten (4), der derart in dem Gehäuse angeordnet ist, dass der Schlitten aus dem Gehäuse herausgezogen werden kann, und
eine Implantathalterung (5), die derart auf dem Schlitten angeordnet ist, dass die Implantathalterung der UV-Lampe in dem Gehäuse zugewandt ist,
wobei die Implantathalterung (5) einen Implantatträger (7), auf dem die Implantate (10) linear angeordnet sind, und einen Auflagetisch (6) beinhaltet, auf dem der Implantatträger lösbar angeordnet ist,
**dadurch gekennzeichnet, dass**
die Implantathalterung eine Bestätigungseinheit (25) für einen effektiven Bestrahlungsbereich beinhaltet, die konfiguriert ist, um einen effektiven Bestrahlungsbereich (X) eines aus der UV-Lampe emittierten UV-Lichtstrahls zu bestimmen,
wobei die Bestätigungseinheit (25) für den effektiven Bestrahlungsbereich Folgendes beinhaltet:
einen auf der Implantathalterung gebildeten ersten Index (20), der konfiguriert ist, um eine Spannweite (Xa) des effektiven Bestrahlungsbereichs (X) in einer Längsrichtung des effektiven Bestrahlungsbereichs zu definieren, und
einen auf einem auf dem Auflagetisch bereitgestellten Griff (8) gebildeten zweiten Index (21), der konfiguriert ist, um eine Spannweite (Xb) des effektiven Bestrahlungsbereichs in einer Vertikalrichtung des effektiven Bestrahlungsbereichs zu definieren.

2. UV-Bestrahlungseinrichtung für Implantate nach Anspruch 1, wobei es sich bei dem ersten Index (20) der Bestätigungseinheit für den effektiven Bestrahlungsbereich, der konfiguriert ist, um die Spannbreite (Xa) des effektiven Bestrahlungsbereichs in der Längsrichtung des effektiven Bestrahlungsbereichs zu definieren, um eine Länge eines Strukturelements (7; 7a) des Implantatträgers in der Längsrichtung des effektiven Bestrahlungsbereichs (X) handelt.

3. UV-Bestrahlungseinrichtung für Implantate nach Anspruch 1, wobei es sich bei der Bestätigungseinheit für den effektiven Bestrahlungsbereich um ein Plattenelement (22) handelt, das lösbar an dem Auflagetisch angebracht ist, und das Plattenelement eine in diesem gebildete Öffnung (23) aufweist, die dem effektiven Bestrahlungsbereich (X) entspricht.

4. UV-Bestrahlungseinrichtung für Implantate nach Anspruch 1, wobei es sich bei der Bestätigungseinheit für den effektiven Bestrahlungsbereich um ein Plattenelement (22) handelt, das Licht durchlässt und lösbar an dem Auflagetisch (6) angebracht ist, und das Plattenelement einen auf diesem gebildeten Index aufweist, der dem effektiven Bestrahlungsbereich (X) entspricht.

## Revendications

1. Dispositif d'irradiation d'ultraviolets pour des implants (10), apte à éliminer des implants un contaminant organique avec un faisceau ultraviolet, comprenant :
une lampe ultraviolette (3) disposée dans un boîtier (1) ;
un chariot (4) disposé dans le boîtier de telle sorte que le chariot puisse être retiré du boîtier ; et
un organe de maintien (5) des implants disposé sur le chariot de telle sorte que l'organe de maintien des implants soit disposé face à la lampe ultraviolette dans le boîtier,
l'organe de maintien (5) comprenant un support (7) pour des implants sur lequel les implants (10) sont disposés linéairement, et une table de support (6) sur laquelle le support d'implants est monté de manière détachable,
***caractérisé en ce que***
l'organe de maintien des implants comprend une unité (25) de confirmation d'une zone d'irradiation effective qui est configurée pour déterminer une zone (X) d'irradiation effective d'un rayon ultraviolet émis par la lampe ultraviolette,
l'unité (25) de confirmation de la zone d'irradiation effective comprenant :
un premier repère (20) formé sur l'organe de maintien des implants et configuré pour définir une plage (Xa) de la zone (X) d'irradiation effective dans une direction longitudinale de la zone d'irradiation effective ; et
un second repère (21) formé sur une poignée (8) présente sur la table de support et configuré pour définir une plage (Xb) de la zone d'irradiation effective dans une direction verticale de la zone d'irradiation effective.

2. Dispositif d'irradiation d'ultraviolets pour des implants selon la revendication 1, dans lequel le premier repère (20) de l'unité de confirmation de la zone d'irradiation effective configuré pour définir la plage (Xa) de la zone (X) d'irradiation effective dans la direction longitudinale de la zone d'irradiation effective est une longueur d'un élément structural (7 ; 7a) du support d'implants dans la direction longitudinale de la zone (X) d'irradiation effective.

3. Dispositif d'irradiation d'ultraviolets pour des implants selon la revendication 1, dans lequel l'unité de confirmation de la zone d'irradiation effective est un élément en forme de plaque (22) qui est fixé de manière détachable à la table de support, et l'élément en forme de plaque présente une ouverture (23) qui y est percée, qui correspond à la zone (X) d'irradiation effective.

4. Dispositif d'irradiation d'ultraviolets pour des implants selon la revendication 1, dans lequel l'unité de confirmation de la zone d'irradiation effective est un élément en forme de plaque (22) qui transmet la lumière et qui est fixé de manière détachable à la table de support (6), et l'élément en forme de plaque présente un repère qui y est formé, qui correspond à la zone d'irradiation effective (X).
